# EUROPEAN PATENT SPECIFICATION

(11) **EP 2 875 798 B1**
(45) Date of publication and mention of the grant of the patent: **01.03.2017**
(21) Application number: 14275247.6
(22) Date of filing: 25.11.2014
(51) Int. Cl.: A61F 2/90, D04C 1/06

(54) **BRAIDED STENT**
GEFLOCHTENER STENT
STENT TRESSÉ

(30) Priority: 26.11.2013 US 201361909050 P
(43) Date of publication of application: 27.05.2015
(60) Divisional application: 17150892.2
(73) Proprietor: Cook Medical Technologies LLC, Bloomington, IN 47404 (US)
(72) Inventor: Milner, Keith, West Lafayette, IN Indiana 47906 (US); Sherman, Sara Marie, Lafayette, IN Indiana 47905 (US); Choules, Brian D., Lafayette, IN Indiana 47905 (US)
(74) Representative: Williams Powell

(56) References cited:
- EP-A1- 0 891 752
- WO-A1-02/09617
- WO-A2-00/44309
- DE-A1-102007 012 964
- US-A- 6 019 786
- US-A1- 2010 016 940

## Description

### BACKGROUND

The present invention relates generally to medical devices and more particularly to a stent structure.

Stents have become relatively common devices for treating a number of organs, such as the vascular system, colon, biliary tract, urinary tract, esophagus, trachea and the like. Stents are useful in treating various ailments including blockages, occlusions, narrowing conditions and other related problems that restrict flow through a passageway (generally referred to as a stenosis). Stents are also useful in a variety of other medical procedures including treating various types of aneurysms.

For example, stents may be used to treat numerous vessels in the vascular system, including coronary arteries, peripheral arteries (e.g., carotid, brachial, renal, iliac and femoral), and other vessels. Stents have become a common alternative for treating vascular conditions because stenting procedures are considerably less invasive than other alternatives. As an example, stenoses in the coronary arteries have traditionally been treated with bypass surgery. In general, bypass surgery involves splitting the chest bone to open the chest cavity and grafting a replacement vessel onto the heart to bypass the stenosed artery. However, coronary bypass surgery is a very invasive procedure that is risky and requires a long recovery time for the patient. By contrast, stenting procedures are performed transluminally and do not require open surgery. In fact, open surgery has been shown to be unsuitable in patients with significant comorbities due to a high risk of mortality, morbidity, and trauma associated with this procedure. Thus, stenting reduces recovery time and the risks associated with surgery are minimized.

Many different types of stents and stenting procedures are possible. In general, however, stents are typically designed as tubular support structures that may be inserted percutaneously and transluminally through a body passageway. Typically, stents are made from a structure that wraps around at least a portion of a circumference and are adapted to compress and expand between a smaller and larger diameter. Stents may be self-expanding so that they elastically expand out to a preset larger diameter, or may be balloon-expandable in which the stent is deployed by applying a high pressure to the stent inner surface by a balloon. However, other types of stents are designed to have a fixed diameter and are not generally compressible. Although stents may be made from many types of materials, including non-metallic materials and natural tissues, common examples of metallic materials that may be used to make stents include stainless steel and Nitinol. Other materials may also be used, such as cobalt-chrome alloys, amorphous metals, tantalum, platinum, gold, titanium, polymers and/or compatible tissues. Typically, stents are implanted within an artery or other passageway by positioning the stent within the lumen to be treated and then expanding the stent from a compressed diameter to an expanded diameter. The ability of the stent to expand from a compressed diameter makes it possible to navigate the stent through narrow, tortuous passageways to the area to be treated while the stent is in a relatively small, compressed diameter. Once the stent has been positioned and expanded at the area to be treated, the tubular support structure of the stent contacts and radially supports the inner wall of the passageway. The implanted stent may be used to mechanically prevent the passageway from closing in order to keep the passageway open to facilitate fluid flow through the passageway. Conversely, stents may also be used to support a graft layer to prevent fluid flow through the side walls of the stent. However, these are only some of the examples of how stents may be used, and stents may be used for other purposes as well.

Self-expanding stents are one common type of stent used in medical procedures. Self-expanding stents are increasingly being used by physicians because of their adaptability to a variety of different conditions and procedures. Self-expanding stents are usually made of shape memory materials or other elastic materials that act like a spring. Typical metals used in this type of stent include Nitinol and 304 stainless steel. However, other materials may also be used. To facilitate stent implantation, self-expanding stents are normally installed on the end of a catheter in a low profile, compressed state. The stent is typically retained in the compressed state by inserting the stent into a sheath at the end of the catheter. The stent is then guided to the portion of the vessel to be treated. Once the catheter and stent are positioned adjacent the portion to be treated, the stent is released by pulling, or withdrawing, the sheath rearward. Normally, a step or other feature is provided on the catheter to prevent the stent from moving rearward with the sheath. After the stent is released from the retaining sheath, the stent springs radially outward to an expanded diameter until the stent contacts and presses against the vessel wall. Traditionally, self-expanding stents have been used in areas where the vasculature experiences a variety of motion, trauma and tortuosity. One common area of use for self-expanding stents is peripheral arteries in the vascular system. One advantage of self-expanding stents for peripheral arteries is that traumas from external sources do not permanently deform the stent. As a result, the stent may temporarily deform during unusually harsh traumas and spring back to its expanded state once the trauma is relieved. However, self-expanding stents may be used in many other applications as well.

Balloon-expandable stents are often used to treat stenosis of the coronary arteries but may be used in other treatments as well. Usually, balloon-expandable stents are made from ductile materials that plastically deform relatively easily. In the case of stents made from metal, 316L stainless steel that has been annealed is a common choice for this type of stent. One procedure for implanting balloon-expandable stents involves mounting the stent circumferentially on the balloon of a balloon-tipped catheter and threading the catheter over a guide wire through a vessel passageway to the area to be treated. Once the balloon is positioned at the narrowed portion of the vessel to be treated, the balloon is expanded by pumping saline through the catheter to the balloon. As a result, the balloon simultaneously dilates the vessel and radially expands the stent within the dilated portion. The balloon is then deflated and the balloon-tipped catheter is retracted from the passageway. This leaves the expanded stent permanently implanted at the desired location. Ductile metal lends itself to this type of stent since the stent may be compressed by plastic deformation to a small diameter when mounted onto the balloon. When the balloon is later expanded in the vessel, the stent is once again plastically deformed to a larger diameter to provide the desired radial support structure. Traditionally, balloon-expandable stents have been more commonly used in coronary vessels than in peripheral vessels because of the deformable nature of these stents. One reason for this is that balloon-expandable stents can be precisely sized to a particular vessel diameter and shape since the ductile metal that is used can be plastically deformed to a desired size and shape. In addition, there is minimal risk that a coronary vessel will experience a trauma from an external source that would permanently deform a balloon-expandable stent.

Stents may also be used in combination with other components to treat a number of medical conditions. For example, stent-graft assemblies are commonly used in the treatment of aneurysms. As those in the art well know, an aneurysm is an abnormal widening or ballooning of a portion of an artery. Generally, this condition is caused by a weakness in the blood vessel wall. High blood pressure and atherosclerotic disease may also contribute to the formation of aneurysms. Common types of aneurysms include aortic aneurysms, cerebral aneurysms, popliteal artery aneurysms, mesenteric artery aneurysms, and splenic artery aneurysms. However, it is also possible for aneurysms to form in blood vessels throughout the vasculature. If not treated, an aneurysm may eventually rupture, resulting in internal hemorrhaging. In many cases, the internal bleeding may be so massive that a patient can die within minutes of an aneurysm rupture. For example, in the case of aortic aneurysms, the survival rate after a rupture can be as low as 20%.

Traditionally, aneurysms have been treated with surgery. For example, in the case of an abdominal aortic aneurysm, the abdomen is surgically opened, and the widened section of the aorta is typically dissected longitudinally. A graft material, such as Dacron, is then inserted into the vessel and sutured at each end to the inner wall of the non-widened portions of the vessel. The dissected edges of the vessel may then be overlapped and sutured to enclose the graft material within the vessel. In smaller vessels where the aneurysm forms a balloon-like bulge with a narrow neck connecting the aneurysm to the vessel, the surgeon may put a clip on the blood vessel wall at the neck of the aneurysm between the aneurysm and the primary passageway of the vessel. The clip then prevents blood flow from the vessel from entering the aneurysm.

An alternative to traditional surgery for treating aneurysms is endovascular treatment of the blood vessel with a stent. This alternative involves implanting a stent in the blood vessel across the aneurysm using conventional catheter-based placement techniques. In one type of stent-based aneurysm treatment, the stent is provided with a generally impermeable graft layer that seals the wall of the stent. Thus, the aneurysm is blocked by the graft layer and blood flow is kept within the primary passageway of the blood vessel. In another type of stent-based aneurysm treatment, the wall of the stent remains permeable but blood flowing into the aneurysm through the wall of the stent is restricted by the stent structure and/or graft layer. With a sufficient amount of flow restriction into the aneurysm, blood flow through the aneurysm will slow and the blood will begin to coagulate in the aneurysm. As a result, the aneurysm eventually will fill with biological material, which prevents further blood flow into the aneurysm. Increasingly, treatments using stents and stent-grafts to treat aneurysms and other conditions are becoming preferred since these procedures result in less trauma and a faster recuperation.
WO 02/09617 describes a self-expanding stent with enhanced radial expansion and shape memory.

### SUMMARY

The present invention seeks to provide an improved stent structure.

According to an aspect of the present invention, there is provided a braided stent as specified in claim 1.

A braided stent is described with first and second filaments that are braided with each other. The first and second filaments extend around the stent wall in opposite directions from each other. The stent also includes a third filament that extends spirally only along the direction of the second filaments. The third filament is braided with the first filaments. The number of third filaments is less than the number of first filaments and the number of second filaments. The third filament is also stiffer than the first and second filaments.

In an embodiment, the first, second and third filaments cover 20% to 50% of the tubular form along at least a middle portion of said tubular wall, 50% to 80% of said tubular form is open along said middle portion to allow blood flow therethrough, and said helix angles of said first, second and third filaments changes along said middle portion of said tubular form to decrease the open area of said tubular form.

Other features, aspects and advantages of the teachings herein are described below in connection with the disclosure of the preferred embodiments.

### BRIEF DESCRIPTION OF THE DRAWINGS

Embodiments of the present invention are described below, by way of example only, with reference to the accompanying drawings, in which:
Figure 1 is a side plan view of a braided;
Figure 2 is an enlarged view of a portion of the stent shown in Figure 1;
Figure 3 is a perspective view of a braided stent;
Figure 4 is a side view of another braided stent; and
Figure 5 is a side view of another braided stent.

### DETAILED DESCRIPTION

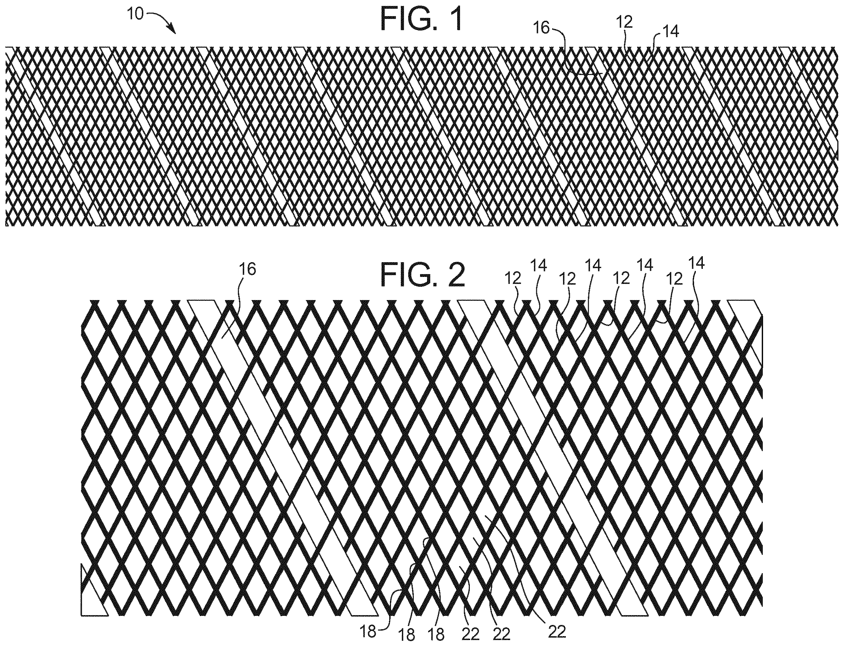

Referring now to the Figures, and particularly to Figures 1-3, a stent 10 is shown, which may be particularly useful for treating an aneurysm, although the stent 10 may also be used for other treatments as well. For example, the stent 10 may be useful in compressing a thrombus against a vessel wall. The stent 10 is preferably self-expanding but may also be balloon expandable. The stent 10 is formed as an braided structure of filaments 12, 14, 16, where the filaments 12, 14, 16 pass over and under each other at the junctions 18. Thus, as shown in Figure 2, the stent 10 has first filaments 12 that extend spirally around the form (hereinafter referred to as the "wall") of the stent 10 in a first direction, and second filaments 14 that extends spirally around the wall of the stent 10 in a second direction. Thus, the first and second filaments 12, 14 spiral around the stent wall in opposite directions, in the sense that the first filaments 12 may extend around in a clockwise direction and the second filaments 14 may extend around in a counterclockwise direction or vice versa. Where the first and second filaments 12, 14 cross 18 each other, the two filaments 12, 14 pass over and under each other. For example, when a first filament 12 crosses a second filament 14, the first filament 12 may pass over the second filament 14. When the first filament 12 crosses the next second filament 14, the first filament 12 will pass under the next second filament 14. Thus, the wall of the stent 10 is formed of a braided structure with the first and second filaments 12, 14 alternatively passing over and then under each other.

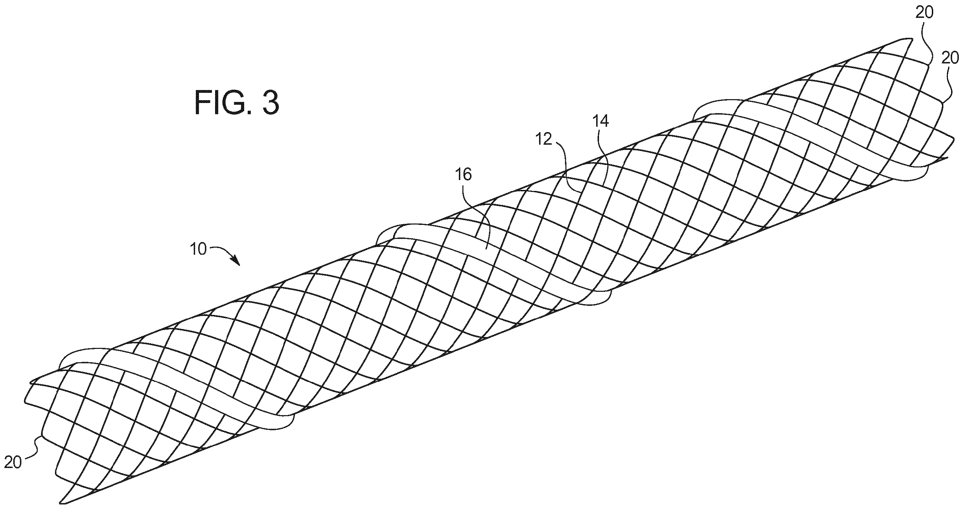

Although there may be variations in the first and second filaments 12, 14, the first and second filaments 12, 14 may be the same as each other in one or more aspects. That is, the braided wall of the stent 10 may have the same number of first filaments 12 as second filaments 14, and the first and second filaments 12, 14 may extend along the same helix angles. Alternatively, there may be more first filaments 12 than second filaments 14 so that the sum of the second filaments 14 and the third filaments 16 equals the number of first filaments 12. Thus, in this embodiment the stent wall may have the same number of filaments extending in opposite directions. The first and second filaments 12, 14 may also have the same diameter as each other and may be made of the same material as each other. As shown in Figure 3, it may also be desirable for the first and second filaments 12, 14 to be a single wire that winds back-and-forth along the length of the stent 10 to form all of the first and second filaments 12, 14. That is, the single wire may extend spirally around the stent wall in the first direction, and then at the end of the stent 10, the wire may be bent 20 to extend spirally back to the first end of the stent 10 along the second direction. As the single wire extends back-and-forth between the ends of the stent 10, the wire passes over and under itself at the crossing junctions 18 as explained above.

The stent 10 also includes one or more third filament 16 that is stiffer than the first and second filaments 12, 14. The third filament 16 is at least 20% stiffer than each of the first and second filaments 12, 14. While increased stiffness of the third filament 16 may be achieved by altering the material properties of the third filament 16 relative to the first and second filaments 12, 14 (e.g., different materials, heat treatments, or other processing), increased stiffness may also be achieved by providing the third filament 16 with a larger diameter than the diameters of the first and second filaments 12, 14. For example, the diameter of the third filament(s) 16 may be at least 4.7% larger than the diameters of the first and second filaments 12, 14, which can provide a stiffness of at least 20% more than that of the first and second filaments. More preferably, the diameters of the first and second filaments 12, 14 is 0.013 mm (0.0005") to 0.038 mm (0.0015"), and the diameter of the third filament 16 is 0.02 mm (0.0008") to 0.076 mm (0.003"). While the stent 10 may have a single third filament 16 as shown in the figures, it is also possible for the stent 10 to have multiple third filaments 16. Like the second filaments 14, the third filament 16 extends spirally around the wall of the stent 10 in the second direction. Thus, the third filament 16 extends in the same direction as the second filaments 14 and is braided with the first filaments 12. Importantly, as described further below, the third filament 16, or filaments 16 where there are multiple third filaments 16, only extends along the second direction and does not extend along the first direction. Thus, the third filament(s) 16 only extends along one spiral direction around stent 10 but does not extend along the opposite spiral direction. Preferably, the second and third filaments 14, 16 extend along the same helix angles. Thus, the second and third filaments 14, 16 are preferably not braided with each other. The helix angles of the first, second and third filaments 12, 14, 16 are all preferably equal to each other, which provides a stent structure 10 that smoothly compresses and expands without binding at the braided junctions 18.

The third filament(s) 16 is also distinguishable from the first and second filaments 12, 14 because the number of third filament(s) 16 is less than the number of first filaments 12 and less than the number of second filaments 14. Thus, the third filaments 16 are the minority of the filaments 14, 16 extending along the second direction. Preferably, the third filaments 16 are less than 25% of the total number of filaments 12, 14, 16 in the stent 10 extending along the first and second directions. It is also possible that the stent 10 may include other filaments for other purposes, such as a filament that is radiopaque to aid in visualization.

Since the third filament 16 extends along only one direction of the stent wall, the ends of the third filament 16 are preferably designed to be atraumatic so that the third filaments 16 do not have sharp ends that may damage tissue. For example, the ends of the third filament 16 may be laser cut to smooth the ends, or could be otherwise treated with welding, coatings, or grinding. The ends may also be bent back toward the first and second directions; however where the third filament 16 may be bent back toward the first direction, the third filament 16 is only bent to form an atraumatic end and does not extend spirally along the first direction. In addition, since there are fewer third filaments 16 than second filaments 14, there will be fewer third filament 16 ends to cause trauma, and treating the third filament 16 ends will be less expensive. For example, where the stent 10 only includes a single third filament 16, the single third filament 16 would only have two ends, which would be relatively straightforward to treat.

The stent 10 may be especially useful in treating aneurysms by allowing blood flow into the aneurysm from the primary passageway through the first, second and third filaments 12, 14, 16. However, because of the close proximity between the first and second filaments 12, 14, blood flow into and out of the aneurysm is restricted compared to before the stent 10 is implanted across the aneurysm. This may cause blood to coagulate within the aneurysm to fill the aneurysm with natural biological material. Thus, the first and second filaments 12, 14 and the third filament 16 may serve different primary purposes in the stent structure 10. That is, the third filament 16 may be primarily intended to provide structural support for expanding the stent structure 10 outward against the vessel wall. Thus, the increased stiffness of the third filament 16 is designed to provide the third filament 16 with greater expansive force compared to the first and second filaments 12, 14. Therefore, the total expansive force of the third filament(s) 16 is preferably greater than the total expansive force of the first and second filaments 12, 14. In addition, because the third filament(s) 16 extends in only one spiral direction, the stent structure 10 is more flexible and more easily and completely conforms to the shape of the vessel. That is, if the third filaments 16 extended in both spiral directions and were braided with each other, the third filaments 16 would bind and pull against each other at the braided junctions 18. Since the third filaments 16 are stiffer, this binding would be considerably more significant than the equivalent binding which may occur between the first and second filaments 12, 14. Thus, if third filaments 16 were braided with each other and extended in both directions, the stent 10 would pull away from the outside curvature of a curved vessel and take the form of an oval cross-section instead of retaining a round cross-section and following the outer curve. Likewise, a stent 10 with braided third filaments 16 may not conform to the inner curve of a curved vessel and may instead buckle along the inner curve, which could disrupt blood flow through the vessel. However, the preferred stent 10 with third filaments 16 extending in only one direction may conform better to the vessel wall while maintaining a circular cross-section without buckling.

By contrast, the first and second filaments 12, 14 may be primarily intended to provide blood flow restriction through the wall of the stent 10. Thus, the first and second filaments 12, 14 may be more flexible than the third filament(s) 16 but may be more numerous. It may also be desirable for the first and second filaments 12, 14 to be smaller in diameter than the third filament(s) 16. Thus, the openings 22 between the first and second filaments 12, 14 may be sized to restrict blood flow into an aneurysm to encourage clotting in the aneurysm. Since the third filament 16 provides structural support for the stent structure 10, the first and second filaments 12, 14 need not be of sufficient stiffness to provide substantial structural support. Thus, the third filament 16 may expand the first and second filaments 12, 14 outward against the vessel wall so that the first and second filaments can perform the function of restricting blood flow through the stent wall. For example, in an aneurysm treatment stent 10, the first, second and third filaments 12, 14, 16 together may cover 20% to 50% of the stent wall along at least a middle portion of the stent 10 where the stent 10 crosses the aneurysm. Thus, 50% to 80% of the stent wall may be open along the middle portion to allow blood flow therethrough.

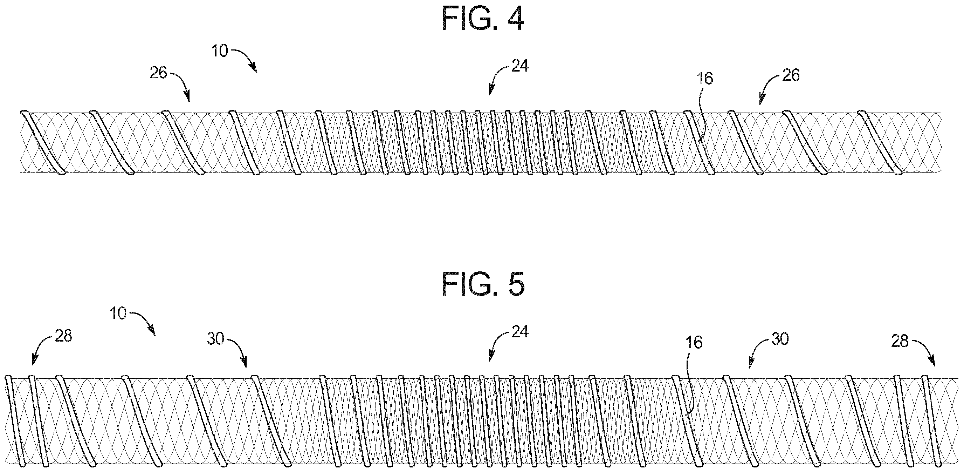

Turning to Figure 4, the helix angles of the first, second and third filaments 12, 14, 16 may change along the length of the stent 10. According to the invention, in an aneurysm treatment stent 10, the helix angles of the first and second filaments 12, 14 changes along the middle portion 24 of the stent wall to decrease the proportion of open area versus area covered by the filaments 12, 14, 16. This provides greater blood restriction along the middle portion 24 where the aneurysm will typically be located and more open area through the filaments 12, 14, 16 at the ends 26 beyond the aneurysm. In order for the stent 10 to smoothly expand and compress with minimal binding at the braided junctions 18, it is preferable for all of the filaments 12, 14, 16 (i.e., the first, second and third filaments 12, 14, 16) to have equally changing helix angles. As shown in Figure 5, it may also be desirable for the helix angle of the third filament 16 to change at the ends of the stent so that adjacent windings are closer together than along the intermediate portions 30 between the ends 28 and between each end 28 and the middle portion 24. This may provide the stent 10 with greater fixation within the vessel at the ends 28. Although the greater fixation is primarily due to the change in the helix angle of the third filament 16, it is preferred that the helix angles of the first and second filaments 12, 14 are equal to the helix angle of the third filament 16 along the ends 28, intermediate portions 30 and middle portion 24.

The skilled person will appreciate that the braiding filaments 12, 14, 16 together form the wall of the stent.

While preferred embodiments of the invention have been described, it should be understood that the invention is not so limited, and modifications may be made without departing from the teachings herein. For example, the method and device may be used to navigate and position a central venous catheter into the venous system with the two described modes of location. Furthermore, the advantages described above are not necessarily the only advantages of the teachings herein, and it is not necessarily expected that all of the described advantages will be achieved with every embodiment of the invention.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

## Claims

1. A braided stent (10), including:
a plurality of first filaments (12), extending spirally around a tubular form along a first direction;
a plurality of second filaments (14) extending spirally around said tubular form along a second direction, said second direction being opposite from said first direction, and said second filaments (14) being braided with said first filaments (12);
a third filament (16) extending spirally around said tubular form along said second direction, and said third filament (16) being braided with said first filaments (12);
wherein said third filament (16) only extends along said second direction, a number of said third filament (16) is less than a number of said first filaments (12) and less than a number of said second filaments (14), and said third filament (16) is at least 20% stiffer than said first filaments (12) and said second filaments (14);
wherein said helix angles of said first (12) and second (14) filaments change along a middle portion (24) of said tubular wall to decrease an open area of said tubular wall along the middle portion, there being more open area at the ends.

2. The braided stent (10) according to claim 1, wherein a diameter of said third filament (16) is at least 4.7% times larger than a diameter of said first filaments (12) and a diameter of said second filaments (14).

3. The braided stent (10) according to claim 1 or 2, wherein a number of said first filaments (12) is equal to said number of said second filaments (14).

4. The braided stent (10) according to claim 1 or 2, wherein a number of said first filaments (12) is equal to a sum of said number of said second filaments (14) and said number of said third filament (16).

5. The braided stent according to any preceding claim, wherein said first (12) and second (14) filaments extend along equal helix angles.

6. The braided stent (10) according to any preceding claim, wherein said second (14) and third (16) filaments extend along equal helix angles.

7. The braided stent (10) according to any preceding claim, wherein a diameter of said first filaments (12) is equal to a diameter of said second filaments (14).

8. The braided stent (10) according to any preceding claim, wherein said first filaments (12) are made from the same material as said second filaments (14).

9. The braided stent (10) according to any preceding claim, wherein said first (12) and second (14) filaments comprise a single wire that is bent (20) at each end.

10. The braided stent (10) according to any preceding claim, comprising a single of said third filament (16) or a plurality of said third filament (16).

11. The braided stent (10) according to any preceding claim, wherein opposing ends of said third filament (16) are atraumatic.

12. The braided stent (10) according to any preceding claim, wherein said first (12), second (14) and third (16) filaments cover 20% to 50% of said tubular form along at least a middle portion of said tubular form.

13. The braided stent (10) according to claim 12, wherein 50% to 80% of said tubular form is open along said middle portion (24) to allow blood flow therethrough.

14. The braided stent (10) according to any preceding claim, wherein said first (12), second (14) and third (16) filaments extend along equal helix angles, and said helix angle of said third filament (16) changes along an end portion (28) of said tubular wall such that adjacent windings are closer together, an intermediate portion (30) between said end portion (28) and said middle portion (24) having helix angles with adjacent windings being farther apart than said end portion (28) and said middle portion (24).

15. The braided stent (10) according to any preceding claim, wherein diameters of said first (12) and second (14) filaments is 0.013 mm (0.0005") to 0.038 mm (0.0015"), and the diameter of the third filament (16) is 0.02 mm (0.0008") to 0.076 mm (0.003").

## Patentansprüche

1. Geflochtener Stent (10), umfassend:
eine Vielzahl von ersten Filamenten (12), die sich spiralförmig um eine röhrenförmige Form entlang einer ersten Richtung erstrecken;
eine Vielzahl von zweiten Filamenten (14), die sich spiralförmig um die röhrenförmige Form entlang einer zweiten Richtung erstrecken, wobei die zweite Richtung zur ersten Richtung entgegengesetzt ist, und wobei die zweiten Filamente (14) mit den ersten Filamenten (12) verflochten sind;
ein drittes Filament (16), das sich spiralförmig um die röhrenförmige Form entlang der zweiten Richtung erstreckt, und wobei das dritte Filament (16) mit den ersten Filamenten (12) verflochten ist;
wobei sich das dritte Filament (16) nur entlang der zweiten Richtung erstreckt, wobei eine Anzahl des dritten Filaments (16) geringer als eine Anzahl der ersten Filamente (12) und geringer als eine Anzahl der zweiten Filamente (14) ist, und wobei das dritte Filament (16) wenigstens 20% steifer als die ersten Filamente (12) und die zweiten Filamente (14) ist;
wobei sich die Steigungswinkel der ersten (12) und zweiten (14) Filamente entlang eines mittleren Abschnitts (24) der röhrenförmigen Wand verändern, um einen offenen Bereich der röhrenförmigen Wand entlang des mittleren Abschnitts zu verringern, wobei an den Enden mehr offener Bereich vorhanden ist.

2. Geflochtener Stent (10) nach Anspruch 1, wobei ein Durchmesser des dritten Filaments (16) wenigstens 4,7% Mal größer als ein Durchmesser der ersten Filamente (12) und eine Durchmesser der zweiten Filamente (14) ist.

3. Geflochtener Stent (10) nach Anspruch 1 oder 2, wobei eine Anzahl der ersten Filamente (12) der Anzahl der zweiten Filamente (14) gleicht.

4. Geflochtener Stent (10) nach Anspruch 1 oder 2, wobei eine Anzahl der ersten Filamente (12) einer Summe der Anzahl der zweiten Filamente (14) und der Anzahl des dritten Filaments (16) gleicht.

5. Geflochtener Stent nach einem der vorhergehenden Ansprüche, wobei sich die ersten (12) und zweiten (14) Filamente entlang gleicher Steigungswinkel erstrecken.

6. Geflochtener Stent (10) nach einem der vorhergehenden Ansprüche, wobei sich die zweiten Filamente (14) und das dritte Filament (16) entlang gleicher Steigungswinkel erstrecken.

7. Geflochtener Stent (10) nach einem der vorhergehenden Ansprüche, wobei ein Durchmesser der ersten Filamente (12) einem Durchmesser der zweiten Filamente (14) gleicht.

8. Geflochtener Stent (10) nach einem der vorhergehenden Ansprüche, wobei die ersten Filamente (12) aus demselben Material gefertigt sind wie die zweiten Filamente (14).

9. Geflochtener Stent (10) nach einem der vorhergehenden Ansprüche, wobei die ersten (12) und zweiten (14) Filamente einen einzelnen Draht umfassen, der an jedem Ende gebogen (20) ist.

10. Geflochtener Stent (10) nach einem der vorhergehenden Ansprüche, umfassend ein einzelnes des dritten Filaments (16) oder eine Vielzahl des dritten Filaments (16).

11. Geflochtener Stent (10) nach einem der vorhergehenden Ansprüche, wobei gegenüberliegende Enden des dritten Filaments (16) atraumatisch sind.

12. Geflochtener Stent (10) nach einem der vorhergehenden Ansprüche, wobei die ersten (12), zweiten (14) und dritten (16) Filamente 20% bis 50% der röhrenförmigen Form zumindest entlang eines mittleren Abschnitts der röhrenförmigen Form bedecken.

13. Geflochtener Stent (10) nach Anspruch 12, wobei 50% bis 80% der röhrenförmigen Form entlang des mittleren Abschnitts (24) offen ist, um Blutfluss dort hindurch zu gestatten.

14. Geflochtener Stent (10) nach einem der vorhergehenden Ansprüche, wobei sich die ersten (12), zweiten (14) und dritten (16) Filamente entlang gleicher Steigungswinkel erstrecken, und wobei sich der Steigungswinkel des dritten Filaments (16) entlang eines Endabschnitts (28) der röhrenförmigen Wand ändert, so dass benachbarte Windungen enger beieinander liegen, wobei ein Zwischenabschnitt (30) zwischen dem Endabschnitt (28) und dem mittleren Abschnitt (24) Steigungswinkel aufweist, wobei benachbarte Windungen weiter auseinander sind als der Endabschnitt (28) und der mittlere Abschnitt (24).

15. Geflochtener Stent (10) nach einem der vorhergehenden Ansprüche, wobei der Durchmesser der ersten (12) und zweiten (14) Filamente 0,013 mm (0,0005 Zoll) bis 0,038 mm (0,0015 Zoll) beträgt, und der Durchmesser des dritten Filaments (16) 0,02 mm (0,0008 Zoll) bis 0,076 mm (0,003 Zoll) beträgt.

## Revendications

1. Stent (10) tressé, comprenant :
une pluralité de premiers filaments (12), qui s'étendent en spirale autour d'une forme tubulaire dans une première direction ;
une pluralité de deuxièmes filaments (14) qui s'étendent en spirale autour de ladite forme tubulaire dans une deuxième direction, ladite deuxième direction étant opposée à ladite première direction, et lesdits deuxièmes filaments (14) étant tressés avec lesdits premiers filaments (12) ;
un troisième filament (16) qui s'étend en spirale autour de ladite forme tubulaire dans ladite deuxième direction, et ledit troisième filament (16) étant tressé avec lesdits premiers filaments (12) ;
dans lequel ledit troisième filament (16) s'étend seulement dans ladite deuxième direction, et ledit troisième filament (16) est inférieur en nombre à un nombre desdits premiers filaments (12) et inférieur à un nombre desdits deuxièmes filaments (14), et ledit troisième filament (16) est au moins 20 % plus raide que lesdits premiers filaments (12) et lesdits deuxièmes filaments (14) ;
dans lequel lesdits angles hélicoïdaux desdits premiers (12) et deuxièmes (14) filaments changent dans une partie (24) médiane de ladite paroi tubulaire pour réduire une zone ouverte de ladite paroi tubulaire le long de la partie médiane, une zone plus ouverte existant au niveau des extrémités.

2. Stent (10) tressé selon la revendication 1, dans lequel un diamètre dudit troisième filament (16) est au moins 4,7 % fois plus grand qu'un diamètre desdits premiers filaments (12) et qu'un diamètre desdits deuxièmes filaments (14).

3. Stent (10) tressé selon la revendication 1 ou 2, dans lequel un nombre desdits premiers filaments (12) est égal audit nombre desdits deuxièmes filaments (14).

4. Stent (10) tressé selon la revendication 1 ou 2, dans lequel un nombre desdits premiers filaments (12) est égal à une somme dudit nombre desdits deuxièmes filaments (14) et dudit nombre desdits troisièmes filaments (16).

5. Stent tressé selon l'une quelconque des revendications précédentes, dans lequel lesdits premiers (12) et deuxièmes (14) filaments s'étendent selon des angles hélicoïdaux égaux.

6. Stent (10) tressé selon l'une quelconque des revendications précédentes, dans lequel lesdits deuxièmes (14) et troisièmes (16) filaments s'étendent selon des angles hélicoïdaux égaux.

7. Système (10) tressé selon l'une quelconque des revendications précédentes, dans lequel un diamètre desdits premiers filaments (12) est égal à un diamètre desdits deuxièmes filaments (14).

8. Stent (10) tressé selon l'une quelconque des revendications précédentes, dans lequel lesdits premiers filaments (12) sont constitués de la même matière que lesdits deuxièmes filaments (14).

9. Stent (10) tressé selon l'une quelconque des revendications précédentes, dans lequel lesdits premiers (12) et deuxièmes (14) filaments comportent un seul fil qui est plié (20) au niveau de chaque extrémité.

10. Stent (10) tressé selon l'une quelconque des revendications précédentes, comportant un seul dudit troisième filament (16) ou une pluralité desdits troisièmes filaments (16).

11. Stent (10) tressé selon l'une quelconque des revendications précédentes, dans lequel les extrémités opposées dudit troisième filament (16) sont atraumatiques.

12. Stent (10) tressé selon l'une quelconque des revendications précédentes, dans lequel lesdits premiers (12), deuxièmes (14) et troisièmes (16) filaments recouvrent 20 à 50 % de ladite forme tubulaire le long d'au moins une partie médiane de ladite forme tubulaire.

13. Stent (10) tressé selon la revendication 12, dans lequel 50 à 80 % de ladite forme tubulaire est ouverte le long de ladite partie (24) médiane pour permettre l'écoulement de sang à travers celle-ci.

14. Stent (10) tressé selon l'une quelconque des revendications précédentes, dans lequel lesdits premiers (12), deuxièmes (14) et troisièmes (16) filaments s'étendent selon des angles hélicoïdaux égaux, et ledit angle hélicoïdal dudit troisième filament (16) change dans une partie (28) d'extrémité de ladite paroi tubulaire de telle sorte que des enroulements adjacents soient plus rapprochés les uns des autres, une partie (30) intermédiaire entre ladite partie (28) d'extrémité et ladite partie (24) médiane ayant des angles hélicoïdaux avec des enroulements adjacents qui sont plus espacés que ladite partie (28) d'extrémité et ladite partie (24) médiane.

15. Stent (10) tressé selon l'une quelconque des revendications précédentes, dans lequel le diamètre desdits premiers (12) et deuxièmes (14) filaments est de 0,013 mm (0,0005 pouce) à 0,038 mm (0,0015 pouce), et le diamètre du troisième filament (16) est de 0,02 mm (0,0008 pouce) à 0,076 mm (0,003 pouce).
